(19) Europäisches Patentamt
European Patent Office
Office européen des brevets

(11) Veröffentlichungsnummer : **0 093 999**
**B1**

(12) **EUROPÄISCHE PATENTSCHRIFT**

(45) Veröffentlichungstag der Patentschrift : **09.09.87**

(51) Int. Cl.⁴ : **A 61 K 9/08**, A 61 K 31/54

(21) Anmeldenummer : **83104329.4**

(22) Anmeldetag : **03.05.83**

# (54) Arzneimittelzubereitungen von Oxicam-Derivaten und Verfahren zu deren Herstellung.

(30) Priorität : **08.05.82 DE 3217315**

(43) Veröffentlichungstag der Anmeldung : **16.11.83 Patentblatt 83/46**

(45) Bekanntmachung des Hinweises auf die Patenterteilung : **09.09.87 Patentblatt 87/37**

(84) Benannte Vertragsstaaten : **AT BE CH DE FR GB IT LI LU NL SE**

(56) Entgegenhaltungen :
**EP-A- 0 002 482**
**FR-M- 2 910**
**NL-A- 7 614 135**
**CHEMICAL ABSTRACTS, Band 81, Nr.12, September 23, 1974, COLUMBUS, OHIO, (US), Seite 342**

(73) Patentinhaber : **GÖDECKE AKTIENGESELLSCHAFT**
**Salzufer 16**
**D-1000 Berlin 10 (DE)**

(72) Erfinder : **Gebhardt, Uwe, Dr.**
**Von-Bayer Strasse 19**
**D-7808 Waldkirch (DE)**
Erfinder : **Augart, Helmut**
**Tannenweg 28a**
**D-7808 Waldkirch (DE)**
Erfinder : **Knecht, Adolf, Dr.**
**Kapellenweg 4a**
**D-7800 Freiburg (DE)**

EP 0 093 999 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Oxicam-Derivate der allgemeinen Formel I

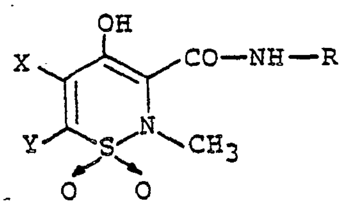

(I)

in welcher R einen heterocyclischen Ring, wie z. B. Pyridin oder 5-Methyl-3-isoxazol bedeutet und X zusammen mit Y einen ankondensierten aromatischen Ring, wie z. B. den Benzol- oder Thiophenring darstellt,

sind sowohl in Wasser als auch in physiologisch unbedenklichen organischen Lösungsmitteln und in deren Mischungen so schlecht löslich, daß pharmazeutische Zubereitungen höherer Konzentration, insbesondere Injektionslösungen, bisher überhaupt noch nicht im Handel sind. Solche mit einem ausreichenden Wirkstoffgehalt konnten bisher nicht realisiert werden.

Oxicam-Derivate der allgemeinen Formel I, wie z. B. Piroxicam (R = Pyridin, X,Y = Benzolring) oder Isoxicam (R = 5-Methyl-3-isoxazol, X,Y = Benzolring) sind hochwirksame Substanzen mit antiinflammatorischer Wirkung, die bisher nur auf oralem Weg appliziert werden. Weitere Substanzen in welchen X,Y einen Thiophenring bedeuten werden in Rheumatherapie 3, 1981, S. 22 beschrieben. Das ebenfalls bekannte Sudoxicam (R = Thiazolyl-2-, X,Y = Benzolring) ist beschrieben in Chem. Abstr. 88 (17) 121216 a.

Da von Mitteln ähnlicher oder gleicher Wirkungsrichtung bekannt ist, daß insbesondere bei entzündlichen und schmerzhaften Erkrankungen des Bewegungsapparates eine lokale Anwendung durch Injektion erherblich rascher wirksam und effektiver sein kann, als eine perorale, systemische Behandlung, besteht ein hohes Bedürfnis nach hochkonzentrierten, injizierbaren Zubereitungen von Wirkstoffen des Oxicam-Typs. Dies trifft insbesondere für die intraartikuläre Applikation zu, die bei nichtsteroiden Mitteln bisher nicht möglich war.

Aus der EP-PS 2482 sind zwar Versuche bekannt, die Löslichkeit von Oxicam-Derivaten durch Salzbildung mit äquimolaren Mengen N-Methyl-D-glucamin zu erhöhen. Hierdurch erreicht man aber nur eine schwache Verbesserung der Löslichkeit.

Auch eigene Versuche zur Lösung des Problems haben gezeigt, daß die Löslichkeit von Verbindungen des Oxicam-Typs der Formel I in Wasser zwar durch stöchiometrische Salzbildung mit Basen an der phenolischen OH-Gruppe in 4-Stellung verbessert werden kann, jedoch reicht die Löslichkeit dieser Salze für die Herstellung konzentrierter Lösungen nicht aus, so daß auf diesem Wege die notwendige Minimierung des Applikationsvolumens nicht erreicht werden konnte.

Speziell bei Oxicam-Derivaten gemäß Formel I besteht darüberhinaus das gravierende Problem der Instabilität ihrer Lösungen. Selbst wenn man solche Verbindungen oder deren Salze einmal in Lösung gebracht hat, neigen diese stark dazu, bereits nach relativ kurzer Zeit Partikel abzuscheiden und einen Teil des Wirkstoffs wieder auszuscheiden. Dies ist natürlich für Injektionslösungen nicht tragbar.

So zeigen Natriumsalze von Isoxicam kurz nach deren Herstellung eine Löslichkeit in Wasser von etwa 24 mg/ml, die jedoch im Verlauf von 4 Monaten durch Ausscheidung des Wirkstoffs aus der Lösung auf etwa 5-6 mg/ml abnimmt. Bei Kaliumsalzen beträgt die anfängliche Löslichkeit in Wasser etwa 30 mg/ml, jedoch sinkt auch diese innerhalb von 4 Monaten auf 20 mg/ml.

Mit Methylglucamin erhält man stöchiometrische Salze des Isoxicams, welche 49 mg Wirkstoff pro Milliliter Lösung enthalten, jedoch waren auch diese instabil und schieden erhebliche Wirkstoffanteile wieder aus. Eigenartig hierbei ist der Befund, daß der Grad der Wirkstoffabscheidung aus der Salz-Lösung in weiten Bereichen von der Konzentration unabhängig ist, so daß sich solche Lösungen auch in niedriger Konzentration für eine parenterale Applikation nicht eignen.

Auch Versuche, die Löslichkeit stöchiometrischer Salze durch Zugabe physiologisch unbedenklicher organischer Lösungsmittel zu erhöhen, führten nicht zum Erfolg, da einmal hierdurch die Löslichkeit nur geringfügig erhöht werden kann und zum anderen dadurch spätere Ausscheidungen des Wirkstoffs aus einmal gebildeten Lösungen nicht verhindert werden.

Aufgabe der vorliegenden Erfindung ist es, hochkonzentrierte und stabile Lösungen sowie Lyophilisate von Wirkstoffen des Oxicam-Typs, insbesondere von Isoxicam, bereitszustellen, welche eine wirksame parenterale und lokale Behandlung entzündlicher Erkrankungen ermöglichen.

Es wurde gefunden, daß man Verbindungen der allgemeinen Formel I überraschend dann in relativ hochkonzentrierte und über mehrere Monate stabile Lösungen überführen kann, wenn man den von der Herstellung von organischen Salzen bekannten stöchiometrischen Basenanteil von 1 : 1 erhöht und gleichzeitig ein physiologisch unbedenkliches organisches, mit Wasser mischbares Lösungsmittel in einer Konzentration ca. 5 bis ca. 70 Vol.%, bevorzugt zwischen 10 und 30 Vol.% zugibt. Der Basenanteil

wird auf einen Anteil von 1 : 1,2 bis 1 : 2 erhöht.

Man erhält dadurch unerwartet und für den Fachmann nicht vorhersehbar hochkonzentrierte Lösungen mit einem Gehalt von bis zu 30 %, bevorzugt 10-20 %, bezogen auf den eingesetzten Wirkstoff, die selbst nach 15 monatiger Lagerung keinerlei Wirkstoffabscheidung zeigen. Erstaunlich ist, daß diese Stabilität auch bei niedrigen Temperaturen voll erhalten bleibt.

Durch das Überwiegen des basischen Anteils steigt der pH-Wert der erhaltenen Lösungen nach dem Verhältnis der Bestandteile auf 9 bis 10 an. Trotz dieses für parenterale Zubereitungen relativ hohen pH-Werts sind, wie Tierversuche gezeigt haben, solche Lösungen bei intramuskulärer, intraarterieller und intravenöser, insbesondere auch bei intraartikulärer Anwendung ausgezeichnet verträglich und verursachen keine Gewebereizungen. Da die Viskosität der Lösungen mit 5 bis 20 mPa · s ziemlich hoch liegen kann, war auch unter diesem Gesichtspunkt eine gewisse Unverträglichkeit zu erwarten. Dies hat sich jedoch auch bei hohen Konzentrationen nicht bestätigt. Trotz dieses überraschenden Befundes sollte der Basenanteil nicht zu hoch liegen, da bei pH-Werten im unphysiologischen Bereich von über 10 mit Gewebeirritationen und Gewebeschäden gerechnet werden muß. Als organische Basen eignen sich besonders Verbindungen der allgemeinen Formel II

$$R—NH—CH_2—(CHOH)_n—CH_2OH$$

in welcher R ein Wasserstoffatom oder eine Alkylgruppe mit 1-6 Kohlenstoffatomen und n die Zahlen 3 oder 4 darstellen.

Bevorzugt sind Verbindungen der Formel II in denen R eine Alkylgruppe mit 1-3 Kohlenstoffatomen bedeutet.

Vertreter der Verbindungen gemäß Formel II sind beispielsweise folgende Zuckeralkohole :

D-Glucamin, N-Methyl-D-Glucamin, N-Hexyl-D-glucamin, N-Butyl-D-glucamin, N-Methyl-D-mannamin, N-Ethyl-D-galactamin, N-Methyl-DL-arabinamin und N-Methyl-L-rhamnamin, N-Methyl-D-xylamin, N-Butyl-D-xylamin.

Die erfindungsgemäßen Zubereitungen sollten eine möglichst niedrige Viskosität aufweisen. Daher haben Verbindungen mit einem niedrigen Molekulargewicht, bei denen demgemäß R eine Methylgruppe und n die Zahl 3 bedeutet, für die Herstellung von Injektionslösungen eine gewisse Präferenz.

Methylglucamin ($R = CH_3$, $n = 4$) ist als physiologisch völlig unbedenklich bekannt und wird bereits in vielen Arzneimittelzubereitungen (z. B. Röntgenkontrastmitteln) verwendet. Darüberhinaus ist es ohne weiteres auf dem Markt verfügbar. Wegen seiner niedrigen Viskosität ist daher Methylglucamin die derzeit günstigste Verbindung der Formel II.

Es versteht sich von selbst, daß die Base erfindungsgemäß frei verfügbar sein muß, so daß der Basenanteil im Falle einer Zugabe von Säuren zur Lösung naturgemäß entsprechend erhöht werden muß.

Als organische Lösungsmittel eignen sich an sich alle mit Wasser mischbaren und physiologisch auch im Hinblick auf eine parenterale Applikation unbedenklich anwendbaren organischen Lösungsmittel, wie Propylenglykol, Polyäthylenglycol und flüssige Säureamide, wie Dimethylformamid und Dimethylacetamid. Besonders bevorzugt sind Polyäthylenglycole der Bezeichnung PEG 200, PEG 300 und PEG 400 sowie Dimethylacetamid. Die Bezeichnung PEG 200, 300, 400 etc. zeigt an, daß ein Polyäthylenglykol mit einem entsprechenden Durchschnittsmolekulargewicht verwendet wird (Nominal average Molecular Weight gemäß USP).

Die Lösungen werden hergestellt, indem man die gewünschte Wirkstoffmenge zusammen mit dem erfindungsgemäßen Überschuß an Base und dem organischen Lösungsmittel auf etwa 40-80 °C, bevorzugt 60-70 °C, erwärmt und anschließend in an sich bekannter Weise zu sterilen Lösungen verarbeitet.

Selbstverständlich eignen sich die Lösungen der Erfindung nicht nur zum Einsatz in parenteral anzuwendenden Arzneimitteln, sondern auch für die Herstellung von Salben, Gelen, ophthalmologischen Präparaten, Tropfen und Säften.

Sie eignen sich insbesondere auch zur Herstellung von Lyophilisaten. In diesem Fall ist das organische Lösungsmittel entbehrlich, da lyophilisierte Produkte erst kurz vor der Anwendung in einem geeigneten Lösungsmittel — in diesem Fall Wasser — gelöst werden müssen und die wässrigen, hochkonzentrierten Lösungen innerhalb von max. 60 Min verwendet werden und somit eine erhöhte Stabilität des Produktes gegen Ausfällungen nicht notwendig ist. Zur Herstellung von gefriergetrockneten Präparaten sollte man allerdings der Basenanteil von 1,35 Mol nicht unterschreiten. Für Injektionslösungen liegt der günstigste Basenanteil zwischen 1,2 Mol und 2 Mol.

Ist es bei hochviskosen Lösungen der Erfindung erwünscht, die Viskosität für die parenterale Verabreichung zu senken, so kann unmittelbar vor der Applikation ohne weiteres die gewünschte Menge Wasser zugegeben werden, ohne daß mit Ausfällungen des Wirkstoffs gerechnet werden muß.

Die Herstellung von Lyophilisaten erfolgt am günstigsten durch Lösen des Wirkstoffs zusammen mit 1,5 bis 2 Mol Base unter Zugabe einer ausreichenden Menge Wasser bei einer Temperatur zwischen 40° und 80 °C. Die klare Lösung wird durch ein Membranfilter von 0,22 μm filtriert, auf kleine Gläschen verteilt, rasch eingefroren und gefriergetrocknet. Der gefriergetrocknete Feststoff löst sich nach Zugabe

der entsprechenden Menge Wasser sofort auf und gibt (auch in Abwesenheit von organischen Lösungsmitteln) eine für mindestens 30 Minuten stabile klare Lösung.

Gegenstand der vorliegenden Erfindung sind daher Arzneimittelzubereitungen mit einem Gehalt an Oxicam-Derivaten der allgemeinen Formel I, dadurch gekennzeichnet, daß sie, bezogen auf den Wirkstoff, eine 1,2 bis 2 molare Menge einer organischen Base der allgemeinen Formel II enthalten.

Ein weiterer Gegenstand der Erfindung sind wässrige Lösungen von Oxicam-Derivaten der allgemeinen Formel I zusammen mit einer 1,2 bis 2 molaren Menge einer Base der allgemeinen Formel II und einem Anteil von 5 bis 70 Vol.% eines physiologisch verträglichen, mit Wasser mischbaren organischen Lösungsmittels.

Ein weiterer Gegenstand der Erfindung ist ein Verfahren zur Herstellung einer Oxicam-Derivate der allgemeinen Formel I enthaltenden Arzneimittelzubereitung, dadurch gegennzeichnet, daß man den Wirkstoff zusammen mit 1,2 bis 2 Mol einer organischen Base der allgemeinen Formel II in einer wässrigen Lösung mit einem Anteil von 5-70 % eines physiologisch verträglichen, mit Wasser mischbaren organischen Lösungsmittels bei einer Temperatur von etwa 40-80 °C löst.

Ein weiterer Gegenstand der Erfindung ist ein Verfahren zur Herstellung einer Oxicam-Derivate enthaltenden Arzneimittelzubereitung der allgemeinen Formel I, dadurch gegennzeichnet, daß man den Wirkstoff zusammen mit einer 1,5 bis 2 molaren Menge einer organischen Base der allgemeinen Formel II bei 40 bis 80 °C in einer zur vollständigen Lösung ausreichenden Menge Wasser löst und die erhaltene Lösung nach Aufteilung in kleine Vorratsgefäße in an sich bekannter Weise rasch einfriert und gefriertrocknet.

Die folgenden Beispiele dienen der näheren Erläuterung der Erfindung :

Beispiel 1

a) 10,75 g Methylglucamin (I) werden in 30 g destilliertem Wasser gelöst. In diese Lösung gibt man nacheinander 15 g Isoxicam (II) und 30 g Polyethylenglycol (III) (PEG 200). Man erwärmt auf 60-70 °C bis zur vollständigen Lösung, füllt nach dem Abkühlen mit destilliertem Wasser auf 100 ml auf und filtriert anschließend durch ein 0,22 µm-Membranfilter. Die so erhaltene klare Lösung wird in 1 ml-Ampullen abgefüllt, die 150 mg Isoxicam enthalten.

In analoger Weise werden folgende Lösungen hergestellt :

| | |
|---|---|
| b) Methylglucamin (I) | 10,75 g |
| Isoxicam (II) | 15,00 g |
| PEG 200 (III) | 20,00 g |
| c) Methylglucamin (I) | 10,75 g |
| Isoxicam (II) | 15,00 g |
| PEG 200 (III) | 10,00 g |
| d) Methylglucamin (I) | 10,75 g |
| Isoxicam (II | 15,00 g |
| PEG 300 (III) | 20,00 g |
| e) Methylglucamin (I) | 10,75 g |
| Isoxicam (II) | 15,00 g |
| PEG 400 (III) | 10,00 g |
| f) Methylglucamin (I) | 10,75 g |
| Isoxicam (II) | 15,00 g |
| Dimethylacetamid (III) | 30,00 g |
| g) Methylglucamin (I) | 10,75 g |
| Piroxicam (II) | 15,00 g |
| PEG 200 (III) | 30,00 g |
| h) Methylglucamin (I) | 10,75 g |
| Piroxicam (II) | 15,00 g |
| Dimethylacetamid (III) | 30,00 g |

Die Beispiele i.) bis r.) werden auf 50 ml aufgefüllt :

| | |
|---|---|
| i) Ethylglucamin (I) | 5,76 g |
| Isoxicam (II) | 7,50 g |
| PEG 200 (III) | 15,00 g |
| j) Ethylglucamin (I) | 6,08 g |
| Isoxicam (II) | 7,50 g |
| PEG 200 (III) | 15,00 g |
| k) Ethylglucamin (I) | 7,02 g |
| Isoxicam (II) | 7,50 g |
| PEG 200 (III) | 15,00 g |
| l) Ethylglucamin (I) | 9,36 g |

| | |
|---|---|
| Isoxicam II | 7,50 g |
| PEG 200 (III) | 15,00 g |
| m) Methylglucamin (I) | 5,38 g |
| Sudoxicam (II) | 7,50 g |
| PEG 200 (III) | 15,00 g |
| n) Ethylglucamin (I) | 5,76 g |
| Piroxicam (II) | 7,50 g |
| PEG 200 (III) | 15,00 g |
| o) Ethylglucamin (I) | 5,76 g |
| Isoxicam (II) | 7,50 g |
| Dimethylacetamid (III) | 15,00 g |
| p) Ethylglucamin (I) | 5,76 g |
| Isoxicam (II) | 7,50 g |
| PEG 400 (III) | 15,00 g |
| q) Ethylglucamin (I) | 5,76 g |
| Isoxicam (II) | 7,50 g |
| PEG 300 (III) | 15,00 g |
| r) n-Propylglucamin (I) | 6,00 g |
| Isoxicam (II) | 7,50 g |
| PEG 200 (III) | 15,00 g |

Beispiel 2

10,75 g Methylglucamin werden in 20 g destilliertem Wasser gelöst. Nach Zugabe von 15 g Isoxicam wird mit PEG 200 auf 100 ml aufgefüllt. Man erwärmt das Gemisch auf 40 °C bis alles aufgelöst ist, filtriert nach dem Abkühlen durch ein 0,22 µm-Filter und füllt in 1 ml-Ampullen ab. In analoger Weise erhält man eine Lösung unter Einsatz folgender Komponenten :

| | |
|---|---|
| Ethylglucamin | 11,29 g |
| Isoxicam | 15,0 g |
| PEG 200 | ad 100 ml. |

Beispiel 3

13,1 g Methylglucamin werden in 50 ml destilliertem Wasser gelöst und die Lösung nach Zugabe von 15,0 g Isoxicam auf 100 ml aufgefüllt. Unter Rühren wird das Gemisch bis auf 80 °C erwärmt, bis alles gelöst ist. Nach dem Abkühlen auf 30-40 °C wird durch ein 0,22 µm-Membranfilter filtriert, jeweils 1 ml der Lösung in Gefriertrocknungsgläschen eingefüllt und bei — 40 °C bis — 50 °C eingefroren und anschließend gefriergetrocknet. Durch Zugabe von 1 ml Wasser kann eine Injektionslösung zubereitet werden, die für mindestens 30 Minuten klar und partikelfrei bleibt.

In analoger Weise erhält man Lyophilisate unter Einsatz folgender Komponenten :

| | |
|---|---|
| b) Ethylglucamin | 13,10 g |
| Isoxicam | 15,0 g |
| c) Ethylglucamin | 14,04 |
| Isoxicam | 15,0 g |

**Patentansprüche**

1. Arzneimittelzubereitungen mit einem Gehalt an Oxicam-Derivaten der allgemeinen Formel I

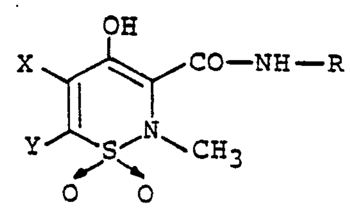

(I)

in welcher R einen heterocyclischen Ring bedeutet und X zusammen mit Y einen ankondensierten aromatischen Ring darstellt,

dadurch gekennzeichnet, daß sie bezogen auf den Wirkstoff, eine 1, 2 bis 2 molare Menge einer organischen Base der allgemeinen Formel II

$$R—NH—CH_2—(CHOH)_n—CH_2OH \quad (II)$$

in welcher R ein Wasserstoffatom oder eine Alkylgruppe mit 1-6 Kohlenstoffatomen und n die Zahlen 3 oder 4 bedeuten enthalten.

2. Arzneimittelzubereitung mit einem Gehalt an mindestens einem Oxicam-Derivat der allgemeinen For I gemäß Anspruch 1, dadurch gekennzeichnet, daß sie in wässriger Lösung einen Anteil von 5 bis 70 Vol.% eines physiologisch verträglichen, mit Wasser mischbaren organischen Lösungsmittels enthält.

3. Arzneimittelzubereitung gemäß Anspruch 1 und 2, dadurch gekennzeichnet, daß sie als Wirkstoff Isoxicam enthält.

4. Arzneimittelzubereitung gemäß Anspruch 1 bis 3, dadurch gekennzeichnet, daß sie als Base Methylglucamin enthält.

5. Arzneimittelzubereitung gemäß Anspruch 2 bis 4, dadurch gekennzeichnet, daß sie als organisches Lösungsmittel Polyethylenglycol oder Dimethylacetamid enthält.

6. Arzneimittelzubereitung nach Anspruch 2-5, dadurch gekennzeichnet, daß die Konzentration des Wirkstoffs gemäß Formel 1 zwischen 10 und 20 % liegt.

7. Arzneimittezubereitung nach Anspruch 2-6, dadurch gekennzeichnet, daß der Anteil an organischem Lösungsmittel zwischen 10 und 30 Gew.% pro Volumen liegt.

8. Arzneimittelzubereitung nach Anspruch 1 dadurch gekennzeichnet, daß sie kein organisches Lösungsmittel und 1,5 bis 2 Mol der organischen Base enthält und als Lyophilisat vorliegt.

9. Verfahren zur Herstellung einer Oxicam-Derivate der allgemeinen Formel I enthaltenden Injektionslösung, dadurch gekennzeichnet, daß man den Wirkstoff zusammen mit einer 1,2 bis 2 molaren Menge einer organischen Base der allgemeinen Formel II in einer wässrigen Lösung mit einem Anteil von 5-70 % eines physiologisch verträglichen, mit Wasser mischbaren organischen Lösungsmittels bei einer Temperatur von etwa 40 bis 80 °C löst.

10. Verfahren zur Herstellung eines Oxicam-Derivate der allgemeinen Formel I enthaltenden Lyophilisats dadurch gekennzeichnet, daß man den Wirkstoff zusammen mit einer 1,5 bis 2 molaren Menge einer organischen Base der allgemeinen Formel II bei 40 bis 80 °C in einer zur vollständigen Lösung ausreichenden Menge Wasser löst und die so erhaltene Lösung in an sich bekannter Weise bei tiefer Temperatur einfriert und gefriertrocknet.

**Claims**

1. Medicinal compositions with a content of oxicam derivatives of the general formula I

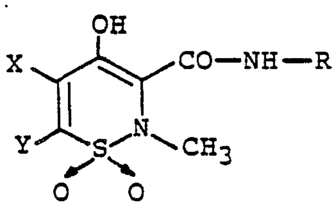

(I)

in wich R signifies a heterocyclic ring and X, together with Y, represents a condensed-on aromatic ring, characterised in that, referred to the active material, it contains a 1.2 to 2 molar amount of an organic base of the general formula II

$$R—NH—CH_2—(CHOH)_n—CH_2OH \quad (II)$$

in which R signifies a hydrogen atom or an alkyl group with 1-6 carbon atoms and n the numbers 3 or 4.

2. Medicinal composition with a content of at least one oxicam derivative of the general formula I according to claim 1, characterised in that it contains, in aqueous solution, a proportion of 5 to 70 vol.% of a physiologically acceptable, water-miscible organic solvent.

3. Medicinal composition according to claim 1 and 2, characterised in that, as active material, it contains isoxicam.

4. Medicinal composition according to claim 1 to 3, characterised in that it contains methylglucamine as base.

5. Medicinal composition according to claim 2 to 4, characterised in that as organic solvent it contains polyethylene glycol or dimethylacetamide.

6. Medicinal composition according to claim 2-5, characterised in that the concentration of the active material according to formula I lies between 10 and 20 %.

7. Medicinal composition according to claim 2-6, characterised in that the proportion of organic solvent lies between 10 and 30 wt.% per volume.

8. Medicinal composition according to claim 1, characterised in that it contains no organic solvent

and 1.5 to 2 mole of the organic base and is present as lyophilisate.

9. Process for the preparation of an injection solution containing oxicam derivatives of the general formula I, characterised in that one dissolves the active material, together with a 1.2 to 2 molar amount of an organic base of the general formula II, in an aqueous solution with a proportion of 5-70 % of a physiologically acceptable, water-miscible organic solvent at a temperature of about 40 to 80 °C.

10. Process for the preparation of a lyophilisate containing oxicam derivatives of the general formula I, characterised in that one dissolves the active material, together with a 1.5 to 2 molar amount of an organic base of general formula II, at 40 to 80 °C. in an amount of water sufficient for complete solution and, in per se known manner, freezes at low temperature the so obtained solution and freeze-dries.

**Revendications**

1. Préparation médicamenteuse renfermant des dérivés de l'oxicam de formule générale I :

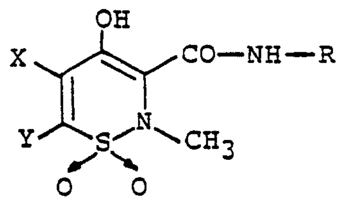

(I)

dans laquelle :

R représente un cycle hétérocyclique ; et

X en même temps que Y représentent un cycle aromatique rapporté par condensation, caractérisée en ce qu'elle contient, par rapport à la substance active, une quantité 1,2 à 2 molaires d'une base organique de formule générale II :

$$R—NH—CH_2—(CHOH)_n—CH_2OH \quad (II)$$

dans laquelle :

R représente un atome d'hydrogène ou un groupe alkyle renfermant de 1 à 6 atomes de carbone ; et n est égal à 3 ou 4.

2. Préparation médicamenteuse renfermant une certaine quantité de dérivés d'oxicam de formule générale I selon la revendication 1, caractérisé en ce qu'elle contient en solution aqueuse une proportion de 5 à 70 % en volume d'un solvant organique physiologiquement compatible, miscible à l'eau.

3. Préparation médicamenteuse selon la revendication 1 ou 2, caractérisée en ce qu'elle contient comme substance active de l'isoxicam.

4. Préparation médicamenteuse selon la revendication 1 à 3, caractérisée en ce qu'elle renferme comme base de la méthylglucamine.

5. Préparation médicamenteuse selon la revendication 2 à 4, caractérisée en ce qu'elle contient comme solvant organique du polyéthylèneglycol ou du diméthylacétamide.

6. Préparation médicamenteuse selon la revendication 2 à 5, caractérisée en ce que la concentration de la substance active selon la formule I est comprise entre 10 et 20 %.

7. Préparation médicamenteuse selon la revendication 2 à 6, caractérisée en ce que la concentration en solvant organique est comprise entre 10 et 30 % en volume.

8. Préparation médicamenteuse selon la revendication 1, caractérisée en ce qu'elle ne contient pas de solvant organique et qu'elle existe sous forme de lyophilisat.

9. Procédé de préparation d'une solution injectable contenant des dérivés de l'oxicam de formule générale I, caractérisé en ce que l'on dissout la substance active en même temps qu'une quantité 1,2 à 2 molaires d'une base organique de formule générale II en solution aqueuse, en même temps qu'une proportion de 5 à 70 % d'un solvant organique physiologiquement compatible, miscible à l'eau, à une température de 40 à 80 °C.

10. Procédé de préparation d'un lyophilisat contenant des dérivés de l'oxicam de formule générale I, caractérisé en ce que l'on dissout la substance active en même temps qu'une quantité 1,5 à 2 molaires d'une base organique de formule générale II à une température de 40 à 80 °C, dans une quantité d'eau suffisante pour une dissolution complète et on congèle la solution ainsi obtenue à basse température, de façon connue en soi, et on la lyophilise.